# EUROPEAN PATENT APPLICATION

(11) **EP 2 829 247 A1**
(43) Date of publication of application: **28.01.2015**
(21) Application number: 13839286.5
(22) Date of filing: 19.09.2013
(51) Int. Cl.: A61B 18/12, A61B 18/00

(54) **SURGERY DEVICE**

(30) Priority: 21.09.2012 US 201261704080 P
(71) Applicant: Olympus Medical Systems Corp., Tokyo 151-0072 (JP)
(72) Inventor: SHIMOMURA Koji, Tokyo 151-0072 (JP)
(74) Representative: Gunzelmann, Rainer
(86) International application number: PCT/JP2013/075259
(87) International publication number: WO 2014/046162

(57) **Abstract**

A surgical apparatus 1 includes a treatment portion 31, a high-frequency output portion 44 or the like that supplies energy such as a high-frequency current, a liquid feeding tube 25, a suction tube 26, two pumps 47 and 49, and a CPU 41 as a control portion. In response to stopping of the supply of energy from the high-frequency output portion 44 or the like, the CPU 41 controls the two pumps to be continuously driven for a predetermined extension time period after the supply of energy stops.

## Description

### Technical Field

The present invention relates to a surgical apparatus, and more particularly to a surgical apparatus that is capable of outputting energy.

### Background Art

A surgical apparatus that uses a surgical treatment instrument is used to perform treatment such as coagulation and dissection of living tissue. Various types of surgical treatment instruments are available, such as instruments that grasp or are brought into contact with living tissue to perform treatment. Further, surgical treatment instruments include, for example, an ultrasound treatment instrument that is capable of ultrasound vibration output, a high-frequency treatment instrument that is capable of high-frequency current output, and in recent years, an energy treatment instrument that is capable of simultaneously outputting ultrasound vibrations and a high-frequency current.

As disclosed in Japanese Patent Application Laid-Open Publication No. 3-85173, a surgical apparatus is also available that performs energy treatment while pouring a liquid onto living tissue.

However, when a surgical operation is performed using the energy treatment instrument, there is a case where bubbles and fragments of tissue remain in the liquid poured onto the living tissue, after the treatment, to deteriorate a field of view when observing a region of the surgical operation by an endoscope.

In the case of the system disclosed in Japanese Patent Application Laid-Open Publication No. 3-85173 as mentioned above, a field of view is secured by proving a sensor for detecting turbidity of a circulating liquid and increasing a feeding amount of the circulating liquid using a detection value of the sensor, but since the sensor for detecting the turbidity is provided in a suction path, there is a time delay to arise a problem that the turbidity of the circulating liquid cannot be removed quickly and appropriately and it takes time for improving the field of view.

Further, according to technology disclosed in Japanese Patent Application Laid-Open Publication No. 7-100203, excised pieces of living tissue are removed by increasing a liquid supply amount of a circulating liquid in response to outputting of treatment energy, but there is a problem that it is difficult to perform the treatment since the liquid supply amount is increased during treatment.

In particular, the techniques disclosed in the two publications are intended to secure an observation field of view in the treatment and it is not taken into account to secure a field of view after the treatment, i.e. after the output of the treatment energy stops, and a favorable field of view cannot be secured quickly after the treatment in the techniques disclosed in the two publications.

Further, if the supply amount of the circulating liquid decreases quickly in response to stopping of the energy output, there is a fear that the living tissue stripped off by the treatment accumulates inside a conduit through which the circulating liquid flows and clogs the conduit.

The present invention has been made in view of the foregoing problems and an object of the present invention is to provide a surgical apparatus capable of securing a favorable field of view of an endoscope quickly after energy treatment by positively removing excised pieces of living tissue and bubbles generated during treatment by increasing a feeding amount after the treatment, and also preventing clogging of a conduit after stopping of energy output.

### Disclosure of Invention

### Means for Solving the Problem

A surgical apparatus according to one aspect of the invention of the present application includes: a treatment portion for treating living tissue that is an object of treatment; an energy generation portion that supplies energy to the treatment portion; a liquid feeding conduit for feeding a liquid; a suction conduit for sucking the liquid; a liquid feeding port for feeding the liquid, the liquid feeding port being provided in the treatment portion and communicating with the liquid feeding conduit; a suction port for sucking the liquid, the suction port being provided in the treatment portion and communicating with the suction conduit; a first pump for supplying the liquid to the liquid feeding conduit, the first pump being driven in response to a start of supply of the energy from the energy generation portion; a second pump for sucking the liquid in the suction conduit, the second pump being driven in response to a start of supply of the energy from the energy generation portion; and a control portion that, in response to stopping of supply of the energy from the energy generation portion, after supply of the energy stops, controls the first pump and the second pump to be continuously driven for a predetermined first time period.

A surgical apparatus according to another aspect of the invention of the present application includes: a treatment portion for treating living tissue that is an object of treatment; an energy generation portion that supplies energy to the treatment portion; a liquid feeding conduit for feeding a liquid; a suction conduit for sucking the liquid; a liquid feeding port for supplying the liquid from the liquid feeding conduit between the living tissue and the treatment portion, the liquid feeding port being provided in the treatment portion; a suction port for sucking the liquid, the suction port being provided in the treatment portion; a first pump for supplying the liquid to the liquid feeding conduit, the first pump being driven from time before a start of supply of the energy from the energy generation portion; a second pump for sucking the liquid in the suction conduit, the second pump being driven from time before a start of supply of the energy from the energy generation portion; and a control portion that, in response to stopping of supply of the energy from the energy generation portion, for a predetermined time period after supply of the energy stops, performs control to drive the first pump to increase a supply amount of the liquid, and to drive the second pump to increase a suction amount of the liquid.

### Brief Description of the Drawings

Fig. 1 is a view for describing a configuration of a surgical apparatus according to a first embodiment of the present invention.
Fig. 2 is a block diagram illustrating the configuration of a power supply unit 12, a liquid feeding unit 13, and a suction unit 14 according to the first embodiment of the present invention.
Fig. 3 is a view illustrating information regarding an extension time period Te corresponding to an energy outputting time period To that is stored in a storage portion 42 according to the first embodiment of the present invention.
Fig. 4 is a flowchart illustrating an example of processing of a CPU 41 that controls a HF output portion 44 and pump driving portions 46 and 48 according to the first embodiment of the present invention.
Fig. 5 is a flowchart illustrating an example of processing of the CPU 41 that controls the HF output portion 44 and the pump driving portions 46 and 48 according to the first embodiment of the present invention.
Fig. 6 is a timing chart of an output signal EOUT for a high-frequency current output and pump driving signals PiOUTs and PsOUTa of pumps 47 and 49 according to the first embodiment of the present invention.
Fig. 7 is a flowchart illustrating an example of processing of the CPU 41 that controls the HF output portion 44 and the pump driving portions 46 and 48 according to Modification 1 of the first embodiment of the present invention.
Fig. 8 is a timing chart of the output signal EOUT for a high-frequency current output and the pump driving signals PiOUTs and PsOUTa of the pumps 47 and 49 according to Modification 1 of the first embodiment of the present invention.
Fig. 9 is a timing chart of the output signal EOUT for a high-frequency current output and the pump driving signals PiOUTs and PsOUTa of the pumps 47 and 49 according to Modification 2 of the first embodiment of the present invention.
Fig. 10 is another timing chart of the output signal EOUT for a high-frequency current output and the pump driving signals PiOUTs and PsOUTa of the pumps 47 and 49 according to Modification 2 of the first embodiment of the present invention.
Fig. 11 is a view for describing the configuration of a surgical apparatus including a resectoscope according to a second embodiment of the present invention.
Fig. 12 is a block diagram illustrating the configuration of a power supply unit 12A, a liquid feeding unit 13A, and a suction unit 14A according to the second embodiment of the present invention.
Fig. 13 is a flowchart illustrating an example of processing of the CPU 41 that controls the HF output portion 44 and the pump driving portions 46 and 48 according to the second embodiment of the present invention.
Fig. 14 is a timing chart of the output signal EOUT for a high-frequency current output and the pump driving signals PiOUTs and PsOUTa of the pumps 47 and 49 according to the second embodiment of the present invention.

### Best Mode for Carrying Out the Invention

Hereunder, the present invention will be described by way of embodiments.

### (First Embodiment)

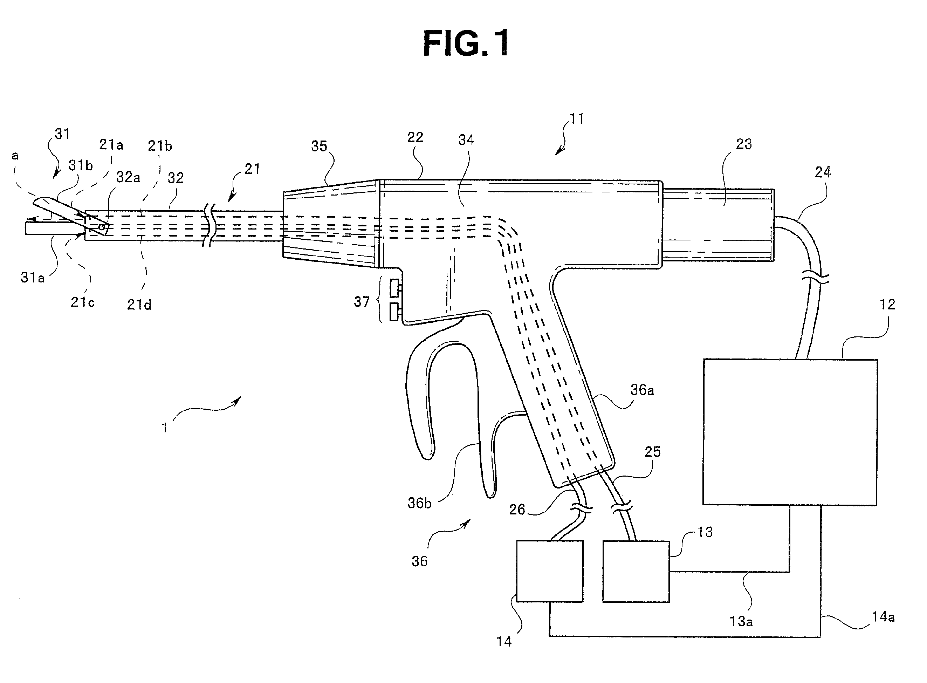

Fig. 1 is a view for describing the configuration of a surgical apparatus according to a first embodiment of the present invention. As shown in Fig. 1, a surgical apparatus 1 is configured to include a treatment instrument 11, a power supply unit 12, a liquid feeding unit 13, and a suction unit 14.

The treatment instrument 11 is a scissors-shaped type surgical treatment instrument that is capable of outputting at least one of ultrasound vibration energy and high-frequency current energy. The treatment instrument 11 is configured to include a treatment unit 21, a handle unit 22, a transducer unit 23, a signal cable 24, a liquid feeding tube 25 and a suction tube 26. The signal cable 24 is connected to the power supply unit 12.

The treatment unit 21 is constituted by a treatment portion 31 for treating living tissue that is an object of treatment, and an elongated sheath portion 32. The treatment portion 31 has a probe 31 a and a movable member 31 b that is a j aw member. The sheath portion 32 is a tubular member through which is inserted a shaft member for performing operations to open and close the probe 31a and the movable member 31b. The movable member 31b is configured to be rotatable around a pin 32a that is provided at a distal end of the sheath portion 32 in response to movement of the shaft member or the like that is caused by a user operation with respect to the handle unit 22. Hence, the distal end portion of the probe 31 a and the movable member 31 b constitute a grasping portion that grasps living tissue.

In addition, an opening portion 21a for feeding physiological saline is provided in a distal end portion of the treatment unit 21, and a tube 21b that is inserted through the inside of the sheath portion 32 is connected to the opening portion 21 a. As shown by a dotted-line arrow a in Fig. 1, the opening portion 21 a and the tube 21b are arranged so that physiological saline can be fed and allowed to drop towards a grasping section between the probe 31a and the movable member 31b of the treatment portion 31. Hence, the opening portion 21 a is a liquid feeding port that communicates with the tube 21b as a liquid feeding conduit provided in the treatment portion 31, and is used for feeding a physiological saline liquid between living tissue and the treatment portion 31.

The proximal end portion of the tube 21b that is also inserted through the inside of the handle unit 22 is connected to the liquid feeding tube 25, and the tube 21b and the liquid feeding tube 25 communicate. As described later, the treatment instrument 11 is configured so that physiological saline that is a liquid that is fed from the liquid feeding unit 13 passes through the liquid feeding tube 25 and the tube 21b and can be emitted from the opening portion 21 a. Hence, the liquid feeding tube 25 and the tube 21b constitute a liquid feeding conduit for feeding a physiological saline liquid.

Further, an opening portion 21c for sucking physiological saline and the like is provided in the distal end portion of the treatment unit 21, and a tube 21 d that is inserted through the inside of the sheath portion 32 is connected to the opening portion 21c. The proximal end portion of the tube 21 d that is also inserted through the inside of the handle unit 22 is connected to the suction tube 26, and the tube 21d and the suction tube 26 communicate. As described later, the treatment instrument 11 is configured so that a liquid such as physiological saline can be sucked from the opening portion 21 c through the suction tube 26 and the tube 21d by the suction unit 14. Hence, the suction tube 26 and the tube 21d constitute a suction conduit for sucking a liquid such as physiological saline. The opening portion 21c is a suction port that is provided in the treatment portion 31 and is used for sucking physiological saline or the like that is inside the tube 21d that is a suction conduit.

The handle unit 22 includes a rotary knob 35 on a distal end side of a tubular body portion 34. The surgeon can change the orientation of the probe 31a of the treatment portion 31 by turning the rotary knob 35 around the axis of the body portion 34.

The transducer unit 23 is mounted at the proximal end portion of the body portion 34. The transducer unit 23 is connected to the probe 31a. The transducer unit 23 contains an ultrasound transducer (not shown) therein, and is configured to be capable of subjecting the probe 31a to ultrasound vibrations.

The body portion 34 has a handle portion 36. The handle portion 36 includes a fixed handle 36a and a movable handle 36b. The handle portion 36 is an operating handle for grasping and holding living tissue. When the surgeon performs an operation so as to bring the movable handle 36b close to the fixed handle 36a, that is, so as to close the handle portion 36, the movable member 31b of the treatment portion 31 is rotated so that living tissue can be grasped between the probe 31a and the movable member 31b.

In addition, a plurality of switches 37 for output operations are provided on the body portion 34. Hence, in a state in which the surgeon operates the treatment instrument 11 while grasping the handle portion 36 to grasp living tissue by means of the distal end portion of the probe 31a and the movable member 31b of the treatment portion 31, by performing an operation to turn on the relevant switch 37, the surgeon can perform treatment by ultrasound vibration output, high-frequency current output, or simultaneous output of ultrasound vibrations and a high-frequency current. That is, the power supply unit 12 that is an energy generation portion generates at least one of a high-frequency current and ultrasound vibrations as energy.

Operation signals of the switches 37 are supplied to the power supply unit 12 through the signal cable 24.

The power supply unit 12 includes a control portion that is, as described later, a control apparatus that performs control of ultrasound vibration output and high-frequency current output as well as liquid feeding and suction in accordance with operations of the switches 37 performed by the surgeon.

The liquid feeding unit 13 is connected by a signal cable 13a to the power supply unit 12. The liquid feeding unit 13 is also connected to the treatment instrument 11 by the liquid feeding tube 25 for feeding liquid, and is configured so that physiological saline from the liquid feeding unit 13 can be fed from the opening portion 21a.

The suction unit 14 is connected by a signal cable 14a to the power supply unit 12. The suction unit 14 is also connected to the treatment instrument 11 by the suction tube 26 for suction, and a configuration is adopted such that physiological saline or the like can be sucked from the opening portion 21c by means of the suction unit 14.

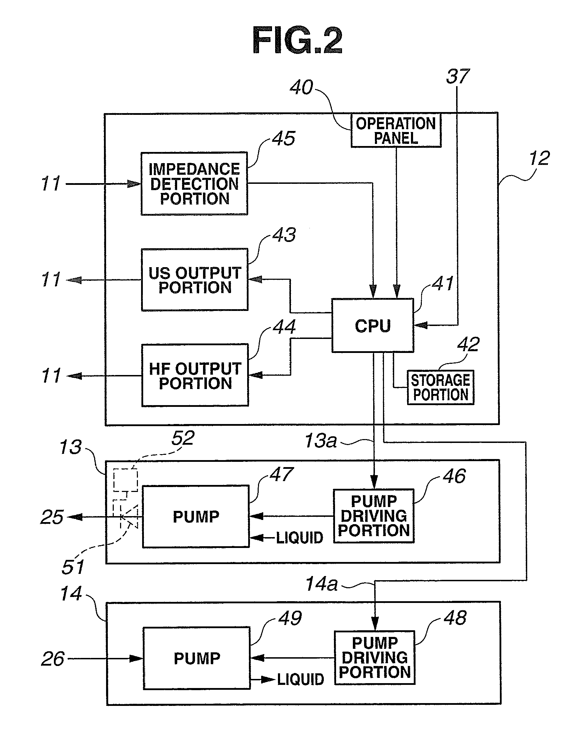

Fig. 2 is a block diagram illustrating the configuration of the power supply unit 12, the liquid feeding unit 13, and the suction unit 14. The power supply unit 12 is a control apparatus that controls the energy output of the treatment instrument 11. The power supply unit 12 includes an operation panel 40 as an operation setting portion, a central processing unit (hereunder, referred to as "CPU") 41 as a control portion, a storage portion 42, an ultrasound output portion (hereunder, referred to as "US output portion") 43 that outputs a driving signal that drives the transducer unit 23 for ultrasound vibration output, a high-frequency output portion (hereunder, referred to as "HF output portion") 44 that outputs a high-frequency current signal for high-frequency current output, and an impedance detection portion 45.

As described above, the CPU 41 performs control of ultrasound vibration output, high-frequency current output, or simultaneous output of ultrasound vibrations and a high-frequency current. The CPU 41 performs the aforementioned control by executing a control program that is stored in the storage portion 42.

The storage portion 42 includes a ROM that stores the control program, a RAM as a working storage area when executing the program, and a rewritable nonvolatile memory that stores information regarding an extension time period that is described later.

The US output portion 43 outputs a driving signal for subjecting the probe 31a to ultrasound vibrations to the treatment instrument 11 through the signal cable 24, based on an ultrasound output control signal from the CPU 41.

The HF output portion 44 outputs a high-frequency current signal for supplying a bipolar high-frequency output to the treatment portion 31 to the treatment instrument 11 through the signal cable 24, based on a high-frequency output control signal from the CPU 41.

Hence, the US output portion 43 and the HF output portion 44 are energy generation portions that supply ultrasound vibrations and a high-frequency current, respectively, as energy to the treatment portion 31.

The impedance detection portion 45 is a circuit for detecting the impedance of living tissue that is grasped between the probe 31 a and the movable member 31b. That is, the impedance detection portion 45 detects the impedance between the two grasping members that grasp living tissue in the treatment portion 31. The impedance detection portion 45 supplies a detection signal corresponding to the impedance between the probe 31a and the movable member 31b to the CPU 41.

Operation signals from the switches 37 are also inputted to the CPU 41. Note that, although in this case an instruction to output energy is performed by the relevant switch 37 being operated by the surgeon as described later, a configuration may also be adopted in which an instruction to output energy is performed by means of a foot switch or the like.

The liquid feeding unit 13 includes a pump driving portion 46 and a pump 47. The pump driving portion 46 is a drive circuit that outputs a driving signal that drives the pump 47, based on a pump driving signal from the CPU 41 that is received through the signal cable 13a. The pump 47 as a first pump is connected to an unshown tank, and is driven based on the driving signal from the pump driving portion 46 to supply physiological saline that is accumulated in the tank to the tube 25.

The discharge capacity of the pump 47 is, for example, 20 ml/min. That is, the pump 47 is a pump for supplying physiological saline to the liquid feeding conduit in order to feed a physiological saline liquid from the opening portion 21a that is a liquid feeding port. That is, the pump 47 is a pump for supplying a liquid to the liquid feeding conduit and is driven in response to the start of the supply of energy from the energy generation portion.

The suction unit 14 includes a pump driving portion 48 and a pump 49. The pump driving portion 48 is a drive circuit that outputs a driving signal that drives the pump 49, based on a pump driving signal from the CPU 41 that is received through the signal cable 14a. The pump 49 as a second pump is connected to an unshown tank, and is driven based on the driving signal from the pump driving portion 48 to discharge physiological saline or the like that was sucked through the tube 26 into the tank (not shown in the drawings). The suction capacity of the pump 49 is, for example, 20 ml/min. That is, the pump 49 is a pump for sucking a liquid in the suction conduit and is driven in response to the start of the supply of energy from the energy generation portion.

The CPU 41 performs control of the US output portion 43 and the HF output portion 44 together with control of the pump driving portions 46 and 48 in accordance with operation signals from the switches 37.

Data with respect to an extension time period Te for which to continue driving of the pumps 47 and 49 and extend circulation after high-frequency output or ultrasound output, or after simultaneously performing high-frequency output and ultrasound output is previously set and stored in the storage portion 42.

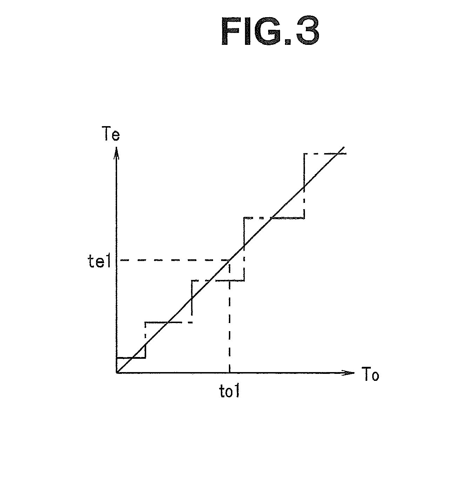

Fig. 3 is a view that illustrates information with respect to the extension time period Te that corresponds to an energy outputting time period To that is stored in the storage portion 42. The extension time period Te is proportional to the energy outputting time period To (or a driving time period of the pumps 47 and 49; the same applies hereunder). Hence, for example, the extension time period Te with respect to the energy outputting time period To is previously set and stored in the storage portion 42 so that the extension time period Te lengthens as the energy outputting time period To increases. The extension time period Te corresponding to the energy outputting time period To is stored in the storage portion 42 in, for example, the form of table data.

Note that a setting with respect to the extension time period Te that corresponds to the energy outputting time period To can be changed by the surgeon or the like on the operation panel 40 of the power supply unit 12.

That is, the extension time period Te is set to a time period that is required in order to achieve a state in which physiological saline or the like that was fed for the purpose of treatment by energy is removed from the operative field.

Note that although in Fig. 3 the extension time period Te is set to proportionally increase in a linear manner relative to the energy outputting time period To, the extension time period Te may also be set to increase in a stepwise manner, as shown by the alternate long and short dashed lines.

As described above, the extension time period Te is previously stored in the storage portion 42, and a setting of the extension time period Te can be changed. Further, a configuration may be adopted in which the extension time period Te is set in accordance with a driving time period of the pumps 47 and 49 that is a time period in which energy is outputted or a time period from when the supply of energy starts until the supply of energy stops.

Next, control of energy output, liquid feeding, and suction according to the present embodiment will be described. Although a case will be described here in which high-frequency output is taken as an example of energy output, the energy output may also be ultrasound output or high-frequency output and ultrasound output that are performed simultaneously.

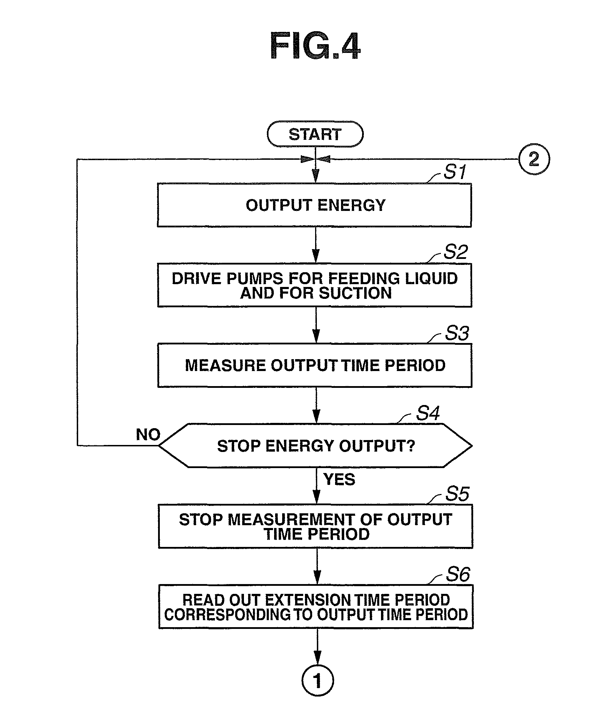

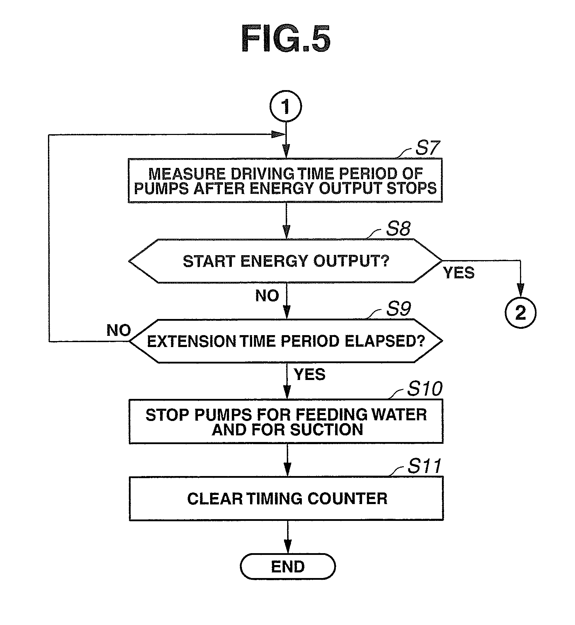

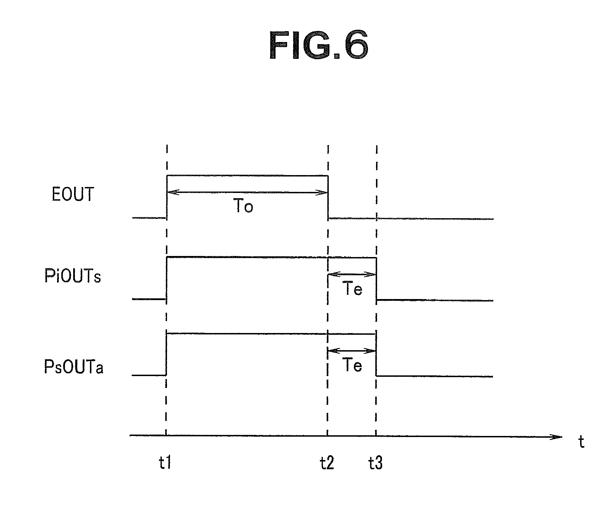

Fig. 4 and Fig. 5 are flowcharts that illustrate an example of processing of the CPU 41 that controls the HF output portion 44 and the pump driving portions 46 and 48. Fig. 6 is a timing chart of an output signal EOUT for a high-frequency current output, and pump driving signals PiOUTs and PsOUTa for the pumps 47 and 49.

The processing in Fig. 4 is executed by the CPU 41 when an instruction to output high-frequency current energy is issued by depressing the relevant switch 37 to turn on the relevant switch 37. As described hereunder, the CPU 41 constitutes a control portion that, in response to stopping of the energy supply from the energy generation portion, controls so as to continue driving of the pumps 47 and 49 for the extension time period Te that is a predetermined time period after the energy supply stops.

When the relevant switch 37 is depressed and an instruction to output energy is received, the CPU 41 performs the instructed energy output (S1). If the energy output is high-frequency current output, the HF output portion 44 is driven to output a high-frequency current of a predetermined or instructed level. Simultaneously with the energy output, the CPU 41 outputs the pump driving signals PiOUTs and PsOUTa to the pump driving portions 46 and 48, respectively, to drive the pumps 47 and 49 (S2). In Fig. 6, the output signal EOUT enters a "high" state at a time t1, and energy output, liquid feeding, and suction are started.

For example, living tissue such as a liver is subjected to treatment by ultrasound output while pouring a liquid such as physiological saline thereon, and suction is also performed with respect thereto so that liquid does not accumulate inside the body.

Simultaneously with the energy output, the CPU 41 also starts to measure the time utilizing a software counter or the like, to thereby measure an outputting time period of the high-frequency current (S3).

Subsequently, the CPU 41 determines if the relevant switch 37 was turned off, that is, if an operation to stop the energy output was performed (S4). If an operation to stop the energy output was not performed (S4: No), the processing returns to S 1. If an operation to stop the energy output was performed (S4: Yes), the CPU 41 stops measurement of the outputting time period (S5).

Next, the CPU 41 calculates the outputting time period with respect to the energy output based on the count value of the counter for measuring the outputting time period, and reads out a value of the extension time period Te stored in the storage portion 42 based on the calculated outputting time period To (S6). For example, in Fig. 3, if the outputting time period To is to 1, a value te1 of the extension time period Te is read out.

The CPU 41 also starts to measure the time utilizing another software counter or the like that is different to the counter for measuring the outputting time period, and thereby measures a driving time period of the pumps 47 and 49 after the energy output stops (S7).

When the pumps 47 and 49 are driving after the energy output stops, the CPU 41 determines whether or not the relevant switch 37 was operated to instruct the start of energy output (S8). If energy output is started (S8: Yes), the processing returns to S1. That is, after the energy output stops, if an instruction to generate energy is received with respect to the HF output portion 44 that is an energy generation portion, the CPU 41 starts the energy output to thus give priority to the operation instruction inputted by the surgeon.

If energy output has not started (S8: No), the CPU 41 determines whether or not the elapsed time period for which the pumps 47 and 49 have been driven since the energy output stopped exceeds the extension time period Te that was read out (S9). If the elapsed time period for which the pumps 47 and 49 have been driven since the energy output stopped does not exceed the extension time period Te that was read out from the storage portion 42 (S9: No), the processing returns to S7 and driving of the pumps 47 and 49 is continued.

When the elapsed time period for which the pumps 47 and 49 have been driven since the energy output stopped exceeds the extension time period Te (S9: Yes), the CPU 41 stops the outputting of the pump driving signals PiOUTs and PsOUTa to the pump driving portions 46 and 48 to thereby stop the pumps 47 and 49 (S10), and clears the two counters used to measure the outputting time period and the extension time period (S11). In Fig. 6, liquid feeding and suction by the pumps 47 and 49 are stopped at a time t3.

That is, in response to stopping of the outputting of energy from the HF output portion 44 that is an energy generation portion, the CPU 41 that is the control portion controls driving of the pumps 47 and 49 so that circulation of physiological saline by the pumps 47 and 49 continues for a predetermined extension time period after the outputting of energy stops. Specifically, in response to stopping of the outputting of energy, the CPU 41 controls so as to continue driving of the pumps 47 and 49 to perform circulation of physiological saline by means of the pumps 47 and 49 for an extension time period that corresponds to the energy outputting time period, and so as to stop the pumps 47 and 49 after performing circulation of physiological saline by means of the pumps 47 and 49 for the predetermined extension time period.

As a result, since circulation is performed during the extension time period Te after energy output, physiological saline that was made turbid by the energy treatment is removed, and the surgeon can swiftly continue the surgical operation.

In addition, a situation in which living tissue that was stripped off by the treatment accumulates inside the tube 21 d and the suction tube 26 and clogs the tube 21 d and the suction tube 26 can be prevented.

Note that, although treatment with respect to the liver was taken as an example in the above description, control of liquid feeding and suction by the above described method is also effective in the case of performing treatment with respect to another organ, such as treatment with respect to the kidney and treatment with respect to the brain.

As described in the foregoing, according to the surgical apparatus 1 of the present embodiment described above, a favorable field of view of an endoscope can be quickly secured after energy treatment, without increasing the liquid supply amount during the treatment, which thus leads to shortening of the time period of the surgical operation. In addition, according to the surgical apparatus 1 of the present embodiment described above, clogging of a conduit after energy output stops can be prevented.

### (Modification 1)

Although according to the above described embodiment, circulation of physiological saline by the pumps 47 and 49 after stopping energy output is performed by the pumps 47 and 49 being driven at the same output level for a predetermined extension time period, according to Modification 1 the pumps 47 and 49 are driven at a high output level for only the predetermined extension time period.

Although the configuration of a surgical apparatus according to Modification 1 is the same as that of the surgical apparatus of the first embodiment that is described above, the processing of the CPU 41 according to Modification 1 is different to the first embodiment. Hence, only the different processing of the CPU 41 according to Modification 1 will be described.

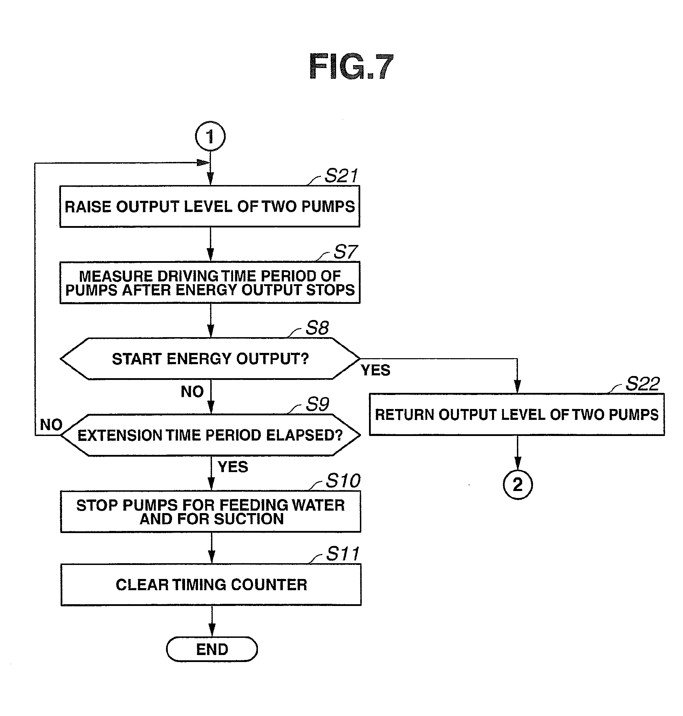

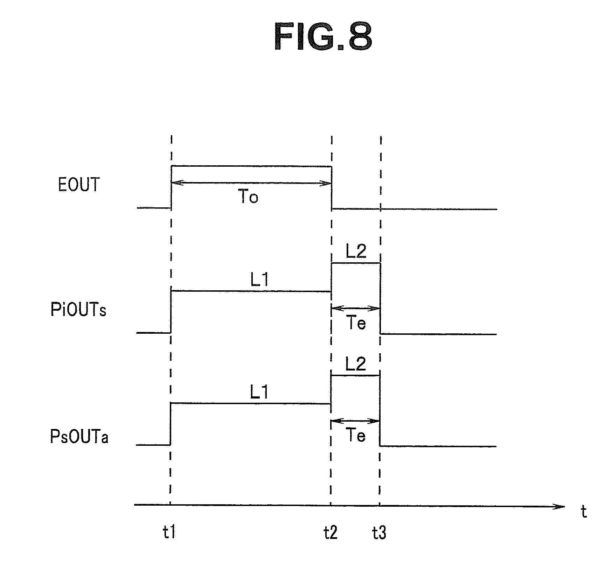

Fig. 4 and Fig. 7 are flowcharts that relate to Modification 1, and illustrate an example of processing of the CPU 41 that controls the HF output portion 44 and the pump driving portions 46 and 48. Fig. 8 is a timing chart of the output signal EOUT for a high-frequency current output and the pump driving signals PiOUTs and PsOUTa of the pumps 47 and 49 according to Modification 1.

When treatment is started, the CPU 41 executes the processing in Fig. 4, and after S6 the CPU 41 raises the output level of the two pumps 47 and 49 and drives the pumps 47 and 49 (S21). For example, when the output level of the pumps 47 and 49 when the pumps 47 and 49 are driven in S2 is L1, in S21 the CPU 41 controls the pump driving portions 46 and 48 so as to change the output level of the pumps 47 and 49 to L2 that is a higher output level than L1 and drive the pumps 47 and 49. The liquid feeding amount and suction amount per unit of time are increased by driving the pumps 47 and 49 at the output level L2 that is higher than L1.

The output level of the pumps 47 and 49 can be changed by changing the driving rotational speed of the pumps. The CPU 41 can change the output level of the pumps 47 and 49 to L2 by controlling the respective pump driving portions 46 and 48 so as to change the driving rotational speed of the pumps from a rotational speed corresponding to the output level L1 to a rotational speed corresponding to the output level L2 to raise the liquid feeding pressure and the suction pressure.

Note that, a configuration may also be adopted so as to increase the liquid feeding amount by additionally providing pressurizing means, without increasing the driving rotational speed of the pump 47 that is the liquid feeding pump. As shown by a dashed line in Fig. 2, a configuration may also be adopted in which a check valve 51 and a second pump 52 are provided on the output side of the pump 47, and in which the second pump 52 is driven when increasing the liquid feeding amount.

After S21, the processing transitions to the processing from S8 to S11.

Note that, when the pumps 47 and 49 are driving after energy output stopped, if the switches 37 are operated and energy output is started (S8: Yes), the CPU 41 returns the output level of the two pumps 47 and 49 to the original output level (S22), and the processing returns to S 1.

As shown in Fig. 8, at a time t2, when energy output is stopped, the output level of the pumps 47 and 49 is raised from L1 to L2, and at a time t3 at which the extension time period Te elapses, the pumps 47 and 49 stop.

Thereafter, if energy output is started again, the pumps 47 and 49 are driven at the first output level, and when energy output stops, driving of the pumps 47 and 49 continues at the second output level that is higher than the first output level for the extension time period Te. That is, the output level of the pumps 47 and 49 during the extension time period Te is higher than the output level of the pumps 47 and 49 while energy is being supplied.

As described above, when energy output stops, the output level of the pumps 47 and 49 is raised and the pumps 47 and 49 are driven for the extension time period Te, and hence a favorable field of view of the endoscope can be secured more quickly.

Note that, although in this case the output level of both of the pumps 47 and 49 is raised, a configuration may also be adopted so as to raise the output level of only one of the pump 47 and the pump 49.

For example, if only the output level of the liquid feeding pump 47 is raised, there is the additional effect that the treatment portion 31 that becomes a high temperature at the time of the treatment can be cooled more quickly.

Further, for example, if only the output level of the suction pump 49 is raised, there is the additional effect that liquid that accumulated inside the body can be quickly sucked and clogging of the tube 21d and the suction tube 26 can be prevented at an earlier stage.

### (Modification 2)

Although in the above described first embodiment and Modification 1 an extension time period of both of the pumps 47 and 49 is the same time period, a configuration may also be adopted in which the extension time period of the pump 49 that is the suction pump is made longer than the extension time period of the liquid feeding pump 47.

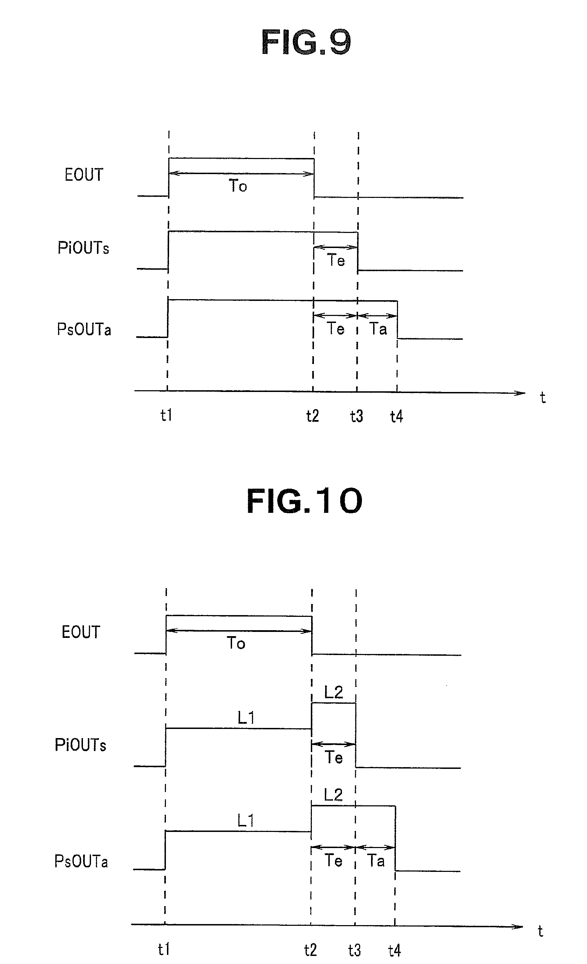

Fig. 9 is a timing chart of an output signal EOUT for the high-frequency current output and pump driving signals PiOUTs and PsOUTa of the pumps 47 and 49 that relates to Modification 2. Fig. 9 illustrates a case where, relative to the case described in the first embodiment, the extension time period of the pump 49 that is the suction pump is made longer than the extension time period of the liquid feeding pump 47.

As shown in Fig. 9, relative to the case described in the first embodiment, the pump driving signal PsOUTa of the pump 49 is outputted for a time period that is longer by a predetermined time period Ta than the extension time period Te in comparison to the pump driving signal PiOUTs of the pump 47.

Fig. 10 is another timing chart of the output signal EOUT for the high-frequency current output and the pump driving signals PiOUTs and PsOUTa of the pumps 47 and 49 that relates to Modification 2. Fig. 10 illustrates a case where, relative to the case described in Modification 1, the extension time period of the pump 49 that is the suction pump is made longer than the extension time period of the liquid feeding pump 47.

As shown in Fig. 10, relative to the case of Modification 1, the pump driving signal PsOUTa of the pump 49 is outputted for a time period that is longer by the predetermined time period Ta than the extension time period Te in comparison to the pump driving signal PiOUTs of the pump 47.

The operations shown in Fig. 9 and Fig. 10 are realized by adopting a configuration so that, in S10 of Fig. 5 and Fig. 7, stopping of the pump 49 is performed after the predetermined time period Ta elapses after stopping the pump 47.

By additionally extending driving of only the pump 49 for the predetermined time period Ta after the extension time period Te elapses and thereby lengthening the driving time period of the pump 49 that is the suction pump in this manner, the CPU 41 can further increase the degree to which clogging of the tube 21d and the suction tube 26 by living tissue that was stripped off by the treatment is prevented.

### (Second Embodiment)

Although the first embodiment relates to a surgical apparatus that uses a sheath-shaped surgical treatment instrument that is capable of outputting two kinds of energy, namely, ultrasound and a high-frequency current, the second embodiment relates to a surgical apparatus that uses a resectoscope as a surgical treatment instrument.

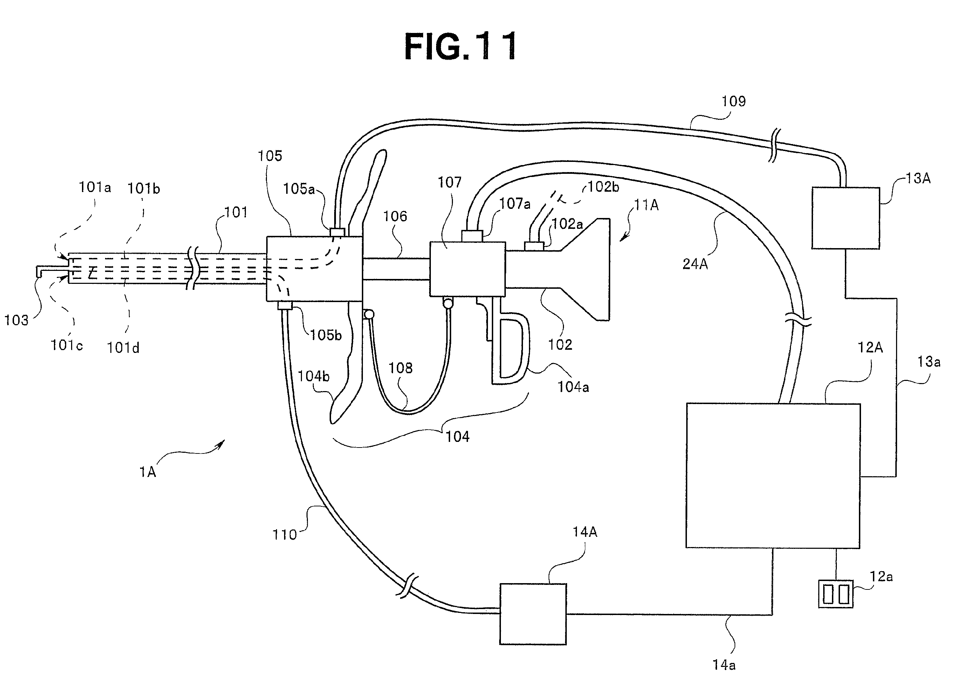

Fig. 11 is a view for describing the configuration of a surgical apparatus including a resectoscope that relates to the second embodiment of the present invention. As shown in Fig. 11, a surgical apparatus 1A is configured to include a resectoscope 11A, a power supply unit 12A, a liquid feeding unit 13A and a suction unit 14A.

The resectoscope 11A includes: a hollow sheath 101, a scope 102 that is inserted through the inside of the sheath 101 and observes a lesioned part, an elongated wire-shaped treatment electrode 103 that treats a lesioned part, a handle portion 104, a sheath connection portion 105, a guide tube 106, and a slider portion 107. One end of a signal cable 24A is connected to a connector 107a provided in the slider portion 107, and the other end is connected to the power supply unit 12A. A light guide connection portion 102a is provided at the proximal end portion of the scope 102, and a light guide 102b is connected to the light guide connection portion 102a.

The treatment electrode 103 that is a treatment portion for treating living tissue that is an object of treatment is connected to a metal pipe (not illustrated in the drawing) that extends to the proximal end side. The proximal end of the metal pipe is connected to an electrode fixing portion inside the slider portion 107.

The sheath connection portion 105 is provided on the proximal end side of the sheath 101. The guide tube 106 is provided so as to protrude from the rear end side of the sheath connection portion 105. An insertion tube of the scope 102 is inserted through the inside of the guide tube 106.

The sheath connection portion 105 and the slider portion 107 are connected by a blade spring 108. The slider portion 107 is constantly urged to the proximal end side (that is, an ocular portion side) of the scope 102 by the blade spring 108.

Hence, when the surgeon manipulates two handles 104a and 104b of the handle portion 104 to bring the two handles 104a and 104b close to each other, an electrode unit that has the treatment electrode 103 in a distal end portion advances to the distal end side along the axial direction inside the sheath 101, and thus the treatment electrode 103 can be caused to protrude from the distal end portion of the sheath 101. Hence, by manipulating the handle portion 104, the surgeon can protrude or retract the treatment electrode 103 that is the treatment portion from the distal end portion of the sheath 101.

A liquid feeding pipe sleeve 105a and a suction pipe sleeve 105b are provided on a side face of the sheath connection portion 105. One end of a liquid feeding tube 109 that is connected to the liquid feeding unit 13A is connected to the liquid feeding pipe sleeve 105a. One end of a suction tube 110 that is connected to the suction unit 14A is connected to the suction pipe sleeve 105b.

In addition, an opening portion 101a for feeding physiological saline is provided in a distal end portion of the sheath 101. The opening portion 101a is connected to one end of a tube 101b that is inserted through the inside of the sheath 101. The opening portion 101 a is a liquid feeding port for feeding physiological saline towards the treatment electrode 103.

The other end of the tube 101b is connected with the liquid feeding pipe sleeve 105a, and thus the tube 101b and the liquid feeding tube 109 that is connected to the liquid feeding unit 13A communicate. As described later, the resectoscope 11A is configured so that physiological saline as a liquid that is fed from the liquid feeding unit 13A can pass through the liquid feeding tube 109 and the tube 101b and be emitted from the opening portion 101a. Hence, the liquid feeding tube 109 and the tube 101b constitute a liquid feeding conduit for feeding physiological saline liquid.

An opening portion 101 c for sucking physiological saline or the like is provided in the distal end portion of the sheath 101, and one end of a tube 101d that is inserted through the inside of the sheath 101 is connected to the opening portion 101c.

The other end of the tube 101d is connected with the suction pipe sleeve 105b, and thus the tube 101d and the suction tube 110 communicate. As described later, the resectoscope 11A is configured so that physiological saline or the like can be sucked by the suction unit 14A from the opening portion 101c through the suction tube 110 and the tube 101d. Hence, the suction tube 110 and the tube 101d constitute a suction conduit for sucking physiological saline liquid or the like. The opening portion 101c is provided in a distal end portion of the sheath 101, and is a suction port for sucking physiological saline or the like into the tube 101d that is a suction conduit.

The power supply unit 12A is a high-frequency power supply apparatus that supplies a high-frequency current to the treatment electrode 103 and to which a feedback current from a return electrode (not illustrated in the drawing) that is provided in the sheath 101 is fed back. Control to turn the power supply to the treatment electrode 103 on or off is performed by turning a foot switch 12a that is connected to the power supply unit 12A on or off.

The liquid feeding unit 13A is connected to the power supply unit 12A by the signal cable 13a. The liquid feeding unit 13A is connected to the liquid feeding pipe sleeve 105a of the resectoscope 11A by the liquid feeding tube 109 for feeding liquid, and is configured to be capable of feeding physiological saline from the liquid feeding unit 13A from the opening portion 101a.

The suction unit 14A is connected to the power supply unit 12A by the signal cable 14a. The suction unit 14A is also connected to the suction pipe sleeve 105b of the resectoscope 11A by the suction tube 110 for sucking, and thus the configuration is such that physiological saline can be sucked from the opening portion 101c by the suction unit 14A.

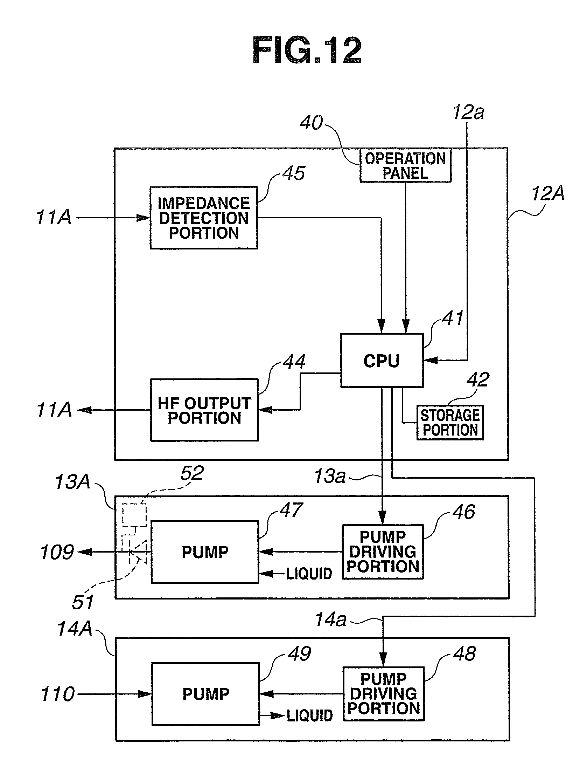

Fig. 12 is a block diagram illustrating the configuration of the power supply unit 12A, the liquid feeding unit 13A, and the suction unit 14A. The power supply unit 12A, the liquid feeding unit 13A, and the suction unit 14A correspond to the power supply unit 12, the liquid feeding unit 13, and the suction unit 14 in the first embodiment, respectively, and the configurations of the power supply unit 12A, the liquid feeding unit 13A, and the suction unit 14A are approximately the same as the configurations shown in Fig. 2.

However, the power supply unit 12A is connected to the resectoscope 11A, and includes the HF output portion 44 that is an energy generation portion that supplies energy to the treatment portion, but does not include the US output portion 43. Further, on and off signals from the foot switch 12a are inputted to the CPU 41. In addition, the pump 47 is connected to the liquid feeding tube 109, and the pump 49 is connected to the suction tube 110.

Hereunder, control of energy output, liquid feeding, and suction according to the present embodiment will be described on the assumption that output control of the resectoscope 11 A, the liquid feeding unit 13A that performs liquid feeding to the resectoscope 11A, and the suction unit 14A that performs suction from the resectoscope 11A is performed by the CPU 41 controlling the HF output portion 44 and the pump driving portions 46 and 48.

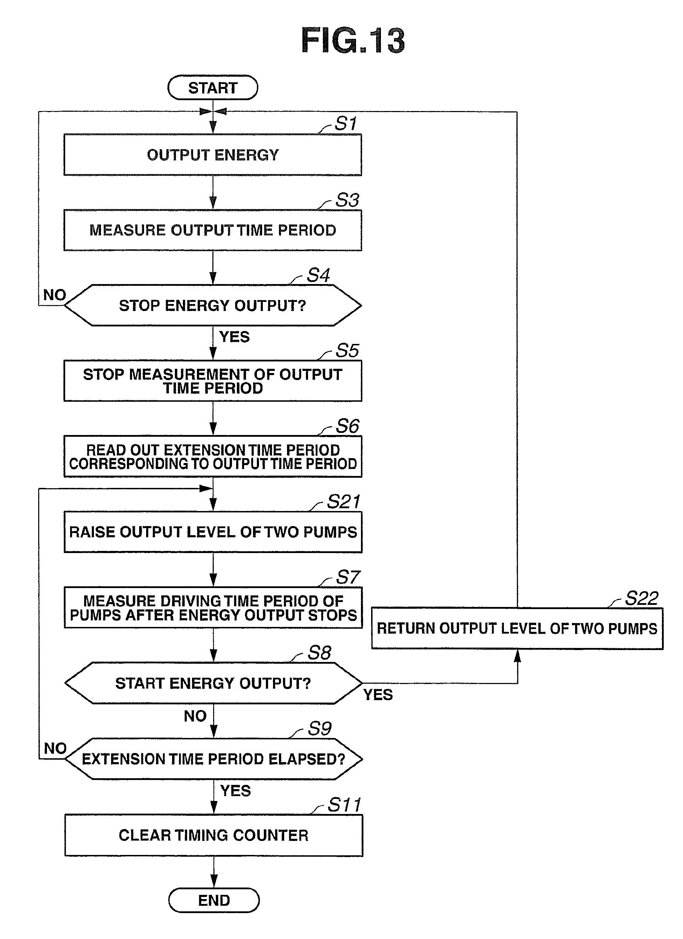

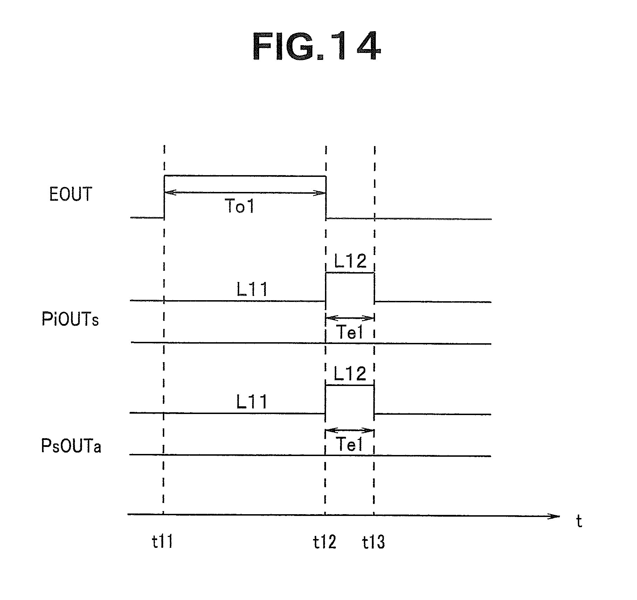

Fig. 13 is a flowchart that illustrates an example of processing of the CPU 41 that controls the HF output portion 44 and the pump driving portions 46 and 48. Fig. 14 is a timing chart with respect to an output signal EOUT for high-frequency current output and pump driving signals PiOUTs and PsOUTa of the pumps 47 and 49. In Fig. 13, operations that are the same as in Fig. 4 and Fig. 5 are denoted by the same reference symbols, and a description of such operations is simplified.

As shown in Fig. 13, when the foot switch 12a is turned on and an instruction to perform energy output is received, the CPU 41 performs the instructed energy output (S1). Regardless of whether energy output is in an on or off state, the liquid feeding unit 13A and the suction unit 14A operate so as to feed a certain amount of liquid and suck a certain amount of liquid, respectively, so that feeding of liquid into the urinary bladder of the patient and drainage of liquid that entered the urinary bladder is performed. That is, according to the present embodiment, the pump 47 is a pump that supplies liquid to the liquid feeding conduit and that is driven before the supply of energy starts from the HF output portion 44 that is the energy generation portion, and the pump 49 is a pump that sucks liquid in the suction conduit and that is driven before the supply of energy starts from the energy generation portion.

In Fig. 14, at a time t11 the output signal EOUT enters a "high" state and energy output is started.

Simultaneously with the energy output, the CPU 41 also starts to measure the time utilizing a software counter or the like, to thereby measure an outputting time period of the high-frequency current (S3).

Subsequently, the CPU 41 determines if the foot switch 12a was turned off, that is, if an operation to stop the energy output was performed (S4). If an operation to stop the energy output was not performed (S4: No), the processing returns to S1. If an operation to stop the energy output was performed (S4: Yes), the CPU 41 stops measurement of the outputting time period (S5).

Next, the CPU 41 calculates the outputting time period with respect to the energy output based on the count value of the counter for measuring the outputting time period, and reads out a value of an extension time period Te1 stored in the storage portion 42 based on the calculated outputting time period To1 (S6). For example, the extension time period Te1 is read out from table data that includes relational data with respect to the relation between an outputting time period and an extension time period as shown in Fig. 3 that is described above.

When treatment is started, the CPU 41 executes the processing in Fig. 13. After S6, the CPU 41 raises the output level of the two pumps 47 and 49 and drives the pumps 47 and 49 (S21). For example, when the output level of the pumps 47 and 49 before energy output starts is L11, in S21 the CPU 41 controls the pump driving portions 46 and 48 so as to change the output level of the pumps 47 and 49 to L12 that is a higher output level than L11 and drive the pumps 47 and 49.

In this case also, changing of the output level of the pumps 47 and 49 can be performed by changing the driving rotational speed of the respective pumps.

After S21, the processing transitions to the processing from S8 to S9, and after S9 the timing counter is cleared (S11).

Note that, when the pumps 47 and 49 are driving at the output level L12 after energy output stopped, if the switches 37 are operated and energy output is started (S8: Yes), the CPU 41 returns the output level of the two pumps 47 and 49 to L11 (S22), and the processing returns to S 1.

As shown in Fig. 14, when the energy output is stopped at a time t12, the output level of the pumps 47 and 49 is raised from L11 to L12. Subsequently, at the timing of a time t13 after the extension time period Te1 elapses, the output level of the pumps 47 and 49 returns to L11. That is, in response to stopping of the energy supply from the energy generation portion, the CPU 41 that is the control portion controls so as to drive the pump 47 to increase the amount of liquid that is supplied and so as to also drive the pump 49 to increase the amount of liquid that is sucked for the predetermined time period Te1 after the energy supply stops.

Thereafter, if energy output stops after energy output was started again, the pumps 47 and 49 are driven at the second output level that is higher than the first output level for the extension time period Te1.

As described above, when energy output is stopped, the output level of the pumps 47 and 49 is raised and the pumps 47 and 49 are driven for the extension time period Te1, and hence a favorable field of view of the scope 102 can be secured more quickly after energy treatment.

Note that, since liquid is constantly circulating during a surgical operation using the resectoscope 11 A, a configuration may also be adopted so as to not only increase the circulation amount after energy output stops, but to also increase the circulation amount only when a switch on the foot switch 12a that is different to the switch for instructing energy output is turned on.

As described in the foregoing, according to the two embodiments described above, a surgical apparatus can be provided that can swiftly secure a favorable field of view of an endoscope after energy treatment by increasing a liquid feeding amount after the treatment to actively remove excised pieces of living tissue and bubbles generated during the treatment, and can also prevent clogging of a conduit after energy output stops.

If the state of a treatment portion cannot be seen or is difficult to see due to bubbles or the like during treatment, the treatment must be interrupted and level of stress felt by the surgeon also increases. However, according to the surgical apparatus in the two embodiments described above, the surgeon can perform a smooth surgical operation without the treatment being interrupted in the aforementioned manner and without an increase in the level of stress felt by the surgeon.

The present invention is not limited to the above described embodiments, and various changes and modifications and the like can be made within a range that does not depart from the spirit and scope of the present invention.

The present application is filed claiming the priority of U.S. Provisional Application No. 61/704,080 filed in U.S. on September 21, 2012, and the above described disclosure is incorporated by reference in the present description, claims and drawings.

## Claims

1. A surgical apparatus, comprising:
a treatment portion for treating living tissue that is an object of treatment;
an energy generation portion that supplies energy to the treatment portion;
a liquid feeding conduit for feeding a liquid;
a suction conduit for sucking the liquid;
a liquid feeding port for feeding the liquid, the liquid feeding port being provided in the treatment portion and communicating with the liquid feeding conduit;
a suction port for sucking the liquid, the suction port being provided in the treatment portion and communicating with the suction conduit;
a first pump for supplying the liquid to the liquid feeding conduit, the first pump being driven in response to a start of supply of the energy from the energy generation portion;
a second pump for sucking the liquid in the suction conduit, the second pump being driven in response to a start of supply of the energy from the energy generation portion; and
a control portion that, in response to stopping of supply of the energy from the energy generation portion, after supply of the energy stops, controls the first pump and the second pump to be continuously driven for a predetermined first time period.

2. The surgical apparatus according to claim 1, wherein output levels of the first pump and the second pump in the predetermined first time period are higher than output levels of the first pump and the second pump during supply of the energy.

3. The surgical apparatus according to claim 1, wherein the energy generation portion generates at least one of a high-frequency current and an ultrasound vibration as the energy.

4. The surgical apparatus according to claim 1, wherein the predetermined first time period is stored in a storage portion and allowed to be set changeably.

5. The surgical apparatus according to claim 1, wherein the predetermined first time period is set in accordance with an outputting time period of the energy or a driving time period of the first pump and the second pump from the start of the supply of the energy to the stopping of the supply of the energy.

6. The surgical apparatus according to claim 1, wherein the control portion further drives only the second pump for a predetermined second time period extensionally after elapse of the predetermined first time period.

7. A surgical apparatus, comprising:
a treatment portion for treating living tissue that is an object of treatment;
an energy generation portion that supplies energy to the treatment portion;
a liquid feeding conduit for feeding a liquid;
a suction conduit for sucking the liquid;
a liquid feeding port for feeding the liquid from the liquid feeding conduit, the liquid feeding port being provided in the treatment portion;
a suction port for sucking the liquid, the suction port being provided in the treatment portion;
a first pump for supplying the liquid to the liquid feeding conduit, the first pump being driven from time before a start of supply of the energy from the energy generation portion;
a second pump for sucking the liquid in the suction conduit, the second pump being driven from time before a start of supply of the energy from the energy generation portion; and
a control portion that, in response to stopping of supply of the energy from the energy generation portion, for a predetermined time period after supply of the energy stops, performs control to drive the first pump to increase a supply amount of the liquid, and to drive the second pump to increase a suction amount of the liquid.

8. The surgical apparatus according to claim 7, wherein the energy generation portion generates a high-frequency current as the energy.

9. The surgical apparatus according to claim 7, wherein the predetermined time period is stored in a storage section and allowed to be set changeably.

10. The surgical apparatus according to claim 7, wherein the predetermined first time period is set in accordance with an outputting time period of the energy or a driving time period of the first pump and the second pump from the start of the supply of the energy to the stop of the supply.

11. The surgical apparatus according to claim 7, wherein the treatment portion is a treatment electrode of a resectoscope.
